# EUROPÄISCHE PATENTANMELDUNG

(11) **EP 4 616 787 A1**
(43) Veröffentlichungstag der Anmeldung: **17.09.2025**
(21) Anmeldenummer: 25161474.9
(22) Anmeldetag: 04.03.2025
(51) Int. Cl.: A61B 3/103, A61B 3/13, A61B 3/14

(54) **VERFAHREN ZUM FOKUSSIEREN EINES OPTISCHEN AUGENBEOBACHTUNGSGERÄTS UND OPTISCHES BEOBACHTUNGSGERÄT**

(30) Priorität: 15.03.2024 DE 102024107408
(71) Anmelder: Carl Zeiss Meditec AG, 07745 Jena (DE)
(72) Erfinder: BEDER, Christian, 73447 Oberkochen (DE); SORG, Benjamin, 73447 Oberkochen (DE); LANG, Thomas, 81379 München (DE)
(74) Vertreter: PATERIS Patentanwälte PartmbB

(57) **Zusammenfassung**

Es wird ein Verfahren zum Einstellen einer Startposition für das Fokussieren eines optischen Augenbeobachtungsgeräts (9) bei Einbringen einer Ophthalmoskopierlupe (1) in den Beobachtungsstrahlengang des optischen Augenbeobachtungsgeräts (9) zur Verfügung gestellt. In dem Verfahren wird das Einbringen der Ophthalmoskopierlupe (1) in den Beobachtungsstrahlengang detektiert, und bei Detektion des Einbringens der Ophthalmoskopierlupe (1) wird die Fokusposition (FP) des optischen Augenbeobachtungsgeräts (9) automatisiert in eine Startposition gebracht. Als Startposition findet eine Fokusposition (FP) des optischen Augenbeobachtungsgeräts (9) Verwendung, in der ein von einer vorbestimmten Ophthalmoskopierlupe (1) aus der Gruppe von in den Beobachtungsstrahlengang des optischen Augenbeobachtungsgeräts (9) einbringbaren Ophthalmoskopierlupen (1, 1', 1") erzeugtes Luftbild (LB) der Retina (15) innerhalb eines Bereiches (B) um die Fokusposition (FP) liegt, dessen Ausdehnung maximal der doppelten Ausdehnung des Tiefenschärfenbereichs (TSB) des optischen Augenbeobachtungsgeräts (9) entspricht.

## Beschreibung

Die vorliegende Erfindung betrifft ein Verfahren zum automatischen Fokussieren eines optischen Augenbeobachtungsgeräts bei Einbringen einer Ophthalmoskopierlupe aus einer Gruppe von Ophthalmoskopierlupen in den Beobachtungsstrahlengang des optischen Augenbeobachtungsgerät. Daneben betrifft die Erfindung ein optisches Augenbeobachtungsgerät.

Zur Ophthalmochirurgie des Hinterabschnitts des Auges werden gerne sog. kontaktlose Visualisierungssysteme eingesetzt, die die frühere Visualisierung mittels eines auf dem Patientenauge aufgesetzten Kontaktglases zurückgedrängt haben. Diese Visualisierungssysteme arbeiten mit sog. Ophthalmoskopierlupen, die nahe über dem Patientenauge in den Beobachtungsstrahlengang eines optischen Augenbeobachtungsgeräts eingebracht werden und ein reelles (wenn auch invertiertes) Luftbild des Augenhintergrundes in einer Zwischenbildebene erzeugen, das dann wiederum mit einem optischen Augenbeobachtungsgerät, bspw. mit einem Operationsmikroskop oder einer Spaltlampe, betrachtet wird. Typische Ophthalmoskopierlupen sind bspw. in US 5 430 506 A oder WO 2010/034502 A2 beschrieben. Um das Luftbild in der Zwischenbildebene betrachten zu können, muss das optische Augenbeobachtungsgerät, das vor dem Einbringen der Ophthalmoskopierlupe auf das Auge selbst fokussiert ist, auf die Zwischenbildebene umfokussiert werden. Da auf jeden Fall der Fokusabstand des optischen Augenbeobachtungsgeräts verkürzt werden muss, wird für das Fokussieren ein bestimmter verkürzter Fokusabstand als Startposition für die Fokussierung auf die Zwischenbildebene eingestellt.

Je nach Applikation finden während einer Operation unterschiedliche, an die Bedürfnisse des Operateurs angepasste Ophthalmoskopierlupen Verwendung. Insbesondere finden in Abhängigkeit von der gewünschten Vergrößerung bzw. des gewünschten Sehwinkels im Auge Ophthalmoskopierlupen mit unterschiedlichen Brechkräften Verwendung. Dies führt dazu, dass die Zwischenbildebenen der Ophthalmoskopierlupen unterschiedlich weit vom Patientenauge entfernt sind. Je nach verwendeter Ophthalmoskopierlupe sind die Zwischenbildebenen, auf die das optische Augenbeobachtungsgerät fokussiert werden muss, dementsprechend unterschiedlich weit vom Hauptobjektiv des optischen Augenbeobachtungsgeräts entfernt, sodass dieses in Abhängigkeit von der verwendeten Ophthalmoskopierlupe auf Zwischenbildebenen in unterschiedlichen Abständen fokussiert werden muss. Bisher wird als Startposition zum Fokussieren des optischen Augenbeobachtungsgeräts eine Fokusposition des optischen Augenbeobachtungsgeräts eingestellt, die zwischen den Fokusebenen der unterschiedlichen Ophthalmoskopierlupen liegt.

Es ist eine erste Aufgabe der vorliegenden Erfindung, ein vorteilhaftes Verfahren zum Einstellen einer Startposition für das Fokussieren eines optischen Augenbeobachtungsgeräts bei Einbringen einer Ophthalmoskopierlupe in den Beobachtungsstrahlengang zur Verfügung zu stellen. Darüber hinaus ist es eine zweite Aufgabe der vorliegenden Erfindung, ein vorteilhaftes optisches Augenbeobachtungsgerät zur Verfügung zu stellen.

Die erste Aufgabe wird gemäß Anspruch 1 durch ein Verfahren zum Einstellen einer Startposition für das Fokussieren eines optischen Augenbeobachtungsgeräts bei Einbringen einer Ophthalmoskopierlupe in den Beobachtungsstrahlengang gelöst, die zweite Aufgabe gemäß Anspruch 13 durch ein optisches Augenbeobachtungsgerät. Die abhängigen Ansprüche enthalten vorteilhafte Ausgestaltungen der Erfindung.

Im erfindungsgemäßen Verfahren zum Einstellen einer Startposition für das Fokussieren eines optischen Augenbeobachtungsgeräts bei Einbringen einer Ophthalmoskopierlupe in den Beobachtungsstrahlengang des optischen Augenbeobachtungsgeräts wird das Einbringen der Ophthalmoskopierlupe in den Beobachtungsstrahlengang detektiert. Bei Detektion des Einbringens der Ophthalmoskopierlupe wird dann die Fokusposition des optischen Augenbeobachtungsgeräts in eine Startposition gebracht, von der aus ein weiteres Fokussieren erfolgen kann, falls dies nötig sein sollte. Als eine Fokusposition soll hierbei die Lage der Fokusebene des optischen Augenbeobachtungsgeräts in Bezug auf eine Bildebene oder Zwischenbildebene angesehen werden, in der sich ein mit dem optischen Augenbeobachtungsgerät zu betrachtendes Bild befindet, insbesondere die Lage der Fokusebene in einer Richtung entlang der optischen Achse des optischen Augenbeobachtungsgeräts. Dabei findet als Startposition eine Fokusposition des optischen Augenbeobachtungsgeräts Verwendung, in der ein von einer vorbestimmten Ophthalmoskopierlupe aus einer Gruppe von in den Beobachtungsstrahlengang des optischen Augenbeobachtungsgeräts einbringbaren Ophthalmoskopierlupen erzeugtes Luftbild der Retina innerhalb eines Bereiches um die Fokusposition liegt, dessen Ausdehnung maximal der doppelten Ausdehnung und vorzugsweise maximal der anderthalbfachen Ausdehnung des Tiefenschärfenbereichs des optischen Augenbeobachtungsgeräts entspricht. Dieser Bereich braucht nicht symmetrisch um die Fokusposition zu sein. Als Tiefenschärfenbereich soll im Rahmen dabei derjenige Bereich um die Fokusposition, in dem Luftbild der Retina von optischen Augenbeobachtungsgeräts scharf abgebildet werden kann, angesehen werden.

Mit dem erfindungsgemäßen Verfahren kann das Luftbild der Retina maximal um die doppelte Ausdehnung des Tiefenschärfenbereiches, vorzugsweise maximal um die anderthalbfache Ausdehnung des Tiefenschärfenbereiches, von der als Startposition verwendeten Fokusposition entfernt liegen, wodurch die Retina in der Startposition bereits zumindest halbwegs scharf abgebildet wird. Auf diese Weise stellt die Startposition zumindest für die vorbestimmte Ophthalmoskopierlupe bereits die richtige Fokusposition dar oder liegt zumindest so nahe bei der richtigen Fokusposition für die vorbestimmte Ophthalmoskopierlupe, dass zumindest grobe Strukturen der Retina bereits erkennbar sind und nur eine geringe Nachfokussierung nötig ist. Zum indest für die vorbestimmte Ophthalmoskopierlupe kann dadurch der weitere Fokussieraufwand minimiert werden.

Besonders vorteilhaft ist es, wenn die Ausdehnung des Bereiches um die Fokusposition maximal der Ausdehnung des Tiefenschärfenbereichs des optischen Augenbeobachtungsgeräts entspricht. Das Luftbild der Retina kann dann maximal so weit von der als Startposition verwendeten Fokusposition entfernt liegen, dass die als Startposition verwendete Fokusposition an dem einen Ende des Tiefenschärfenbereiches liegt und das Luftbild der Retina am andere Ende des Tiefenschärfenbereiches. Mit anderen Worten, als Startposition findet eine Fokusposition des optischen Augenbeobachtungsgeräts Verwendung, bei der das Luftbild der Retina innerhalb des Tiefenschärfenbereiches des optischen Augenbeobachtungsgeräts liegt, so dass bereits in der Startposition eine scharfe Darstellung der Retina gewährleistet ist, wenn in der Startposition die vorbestimmte Ophthalmoskopierlupe zur Anwendung kommt.

Die Gruppe von Ophthalmoskopierlupen kann wenigstens eine der folgenden Ophthalmoskopierlupen umfassen: (i) die am häufigsten in der Behandlung Verwendung findende Ophthalmoskopierlupe, (ii) die als erstes in der Behandlung Verwendung findende Ophthalmoskopierlupe, (iii) diejenige Ophthalmoskopierlupe, welche die höchste Brechkraft aufweist, wobei eine dieser Ophthalmoskopierlupen die vorbestimmte Ophthalmoskopierlupen bildet. Wenn bspw. als vorbestimmte Ophthalmoskopierlupe die am häufigsten oder als erstes in der Behandlung verwendete Ophthalmoskopierlupe bestimmt wird, liegt somit für viele Anwendungsfälle bereits die richtige Fokusposition vor, oder die Fokusposition liegt sehr nahe an der richtigen Fokusposition. Häufig wird diejenige Ophthalmoskopierlupe, welche die höchste Brechkraft aufweist, zuerst verwendet, weil sie den größten Überblick über die Retina ermöglicht. Auf diese Weise liegt für ein Überblickbild bereits die richtige Fokusposition vor, oder die Fokusposition liegt sehr nahe an der richtigen Fokusposition.

Im erfindungsgemäßen Verfahren können aber auch unterschiedliche Startpositionen für eine Anzahl an Ophthalmoskopierlupen aus der Gruppe von Ophthalmoskopierlupen gespeichert sein. Einem Nutzer des optischen Augenbeobachtungsgeräts wird dann die Anzahl an Ophthalmoskopierlupen zur Auswahl einer der Ophthalmoskopierlupen aus dieser Anzahl an Ophthalmoskopierlupen dargeboten. Die vom Nutzer getroffene Auswahl wird erfasst und als Startpositionen findet die für die vom Nutzer ausgewählte Ophthalmoskopierlupe gespeicherte Startposition Verwendung. Auf diese Weise kann für eine Vielzahl von Anwendungsfällen mit der Startposition bereits die richtige Fokusposition herbeigeführt werden oder die Fokusposition zumindest sehr nahe an der richtigen Fokusposition herangeführt werden. Wenn die zur Auswahl stehende Anzahl an Ophthalmoskopierlupen alle in den Beobachtungsstrahlengang des optischen Augenbeobachtungsgeräts einbringbaren Ophthalmoskopierlupen beinhaltet, kann sogar für alle Anwendungsfälle mit der Startposition bereits die richtige Fokusposition herbeigeführt werden oder die Fokusposition zumindest sehr nahe an der richtigen Fokusposition herangeführt werden. Außerdem wird hierdurch eine individuelle Anpassung der Startposition an die vom jeweiligen Chirurgen bevorzugte Ophthalmoskopierlupe oder den aktuellen Bedarf möglich.

Die Lage der Zwischenbildebene für die von den Ophthalmoskopierlupen erzeugten Luftbilder wird durch die Refraktion des Patientenauges beeinflusst. Jedoch werden die allermeisten Hinterabschnittsoperationen in Kombination mit einer Kataraktoperation durchgeführt, d. h. die Augenlinse wird entfernt und durch eine Kunstlinse, eine sogenannte intraokulare Linse (IOL) ersetzt, sofern dies nicht bereits zuvor geschehen ist. Bei Vorhandensein einer intraokularen Linse kann aber von einem rechtsichtigen (emmetropen) Auge oder gegebenenfalls einem leicht kurzsichtigen Auge (intraokulare Linsen werden häufig auf null bis -2 Dioptrien ausgelegt) ausgegangen werden. Die Lagen der Fokusebenen bei einem rechtsichtigen Auge und einem mit -2 Dioptrien kurzsichtigen Auge unterscheiden sich nur um weniger als 1 mm. Als Startposition kann daher eine Fokusposition des Operationsmikroskops Verwendung findet, in der ein von der vorbestimmten Ophthalmoskopierlupe erzeugtes Luftbild der Retina eines emmetropen (rechtsichtigen) Auges innerhalb dieses Bereiches um die Fokusposition liegt. Die Wahrscheinlichkeit, mit einer solchen Startposition bei einem emmetropen Auge bereits ein einigermaßen scharfes Bild zu erhalten, ist hoch. Zumindest ist der Nachfokussierungsaufwand gering. Alternativ kann dem optischen Augenbeobachtungsgerät auch ein Augenrefraktionswert vorgegeben werden und als Startposition eine Fokusposition des optischen Augenbeobachtungsgeräts Verwendung finden, in der ein von der vorbestimmten Ophthalmoskopierlupe erzeugtes Luftbild der Retina eines Auges, das den vorgegebenen Augenrefraktionswert aufweist, innerhalb des Bereiches um die Fokusposition liegt. Die Vorgabe des Augenrefraktionswerts kann beispielsweise im Rahmen der Implantation einer intraokularen Linse vom sogenannten IOL-Master kommen. Als IOL-Master werden dabei Geräte bezeichnet, an denen die intraokulare Linse ausgewählt und die Zielrefraktion, beispielsweise 0 dpt oder -2 dpt, festgelegt wird. Sofern ein solcher IOL-Master mit dem optischen Augenbeobachtungsgerät vernetzt ist, kann dieser die festgelegte Zielrefraktion als vorgegebenen Augenrefraktionswert an das optische Augenbeobachtungsgerät ausgeben. Es besteht aber auch die Möglichkeit, dass einem Nutzer eine Anzahl an Augenrefraktionswerten zur Auswahl eines dieser Augenrefraktionswerte als vorgegebenem Augenrefraktionswert dargeboten wird. Alternativ oder zusätzlich zur Möglichkeit des Auswählens eines Augenrefraktionswertes besteht auch die Möglichkeit, dass im Rahmen des Verfahrens eine Messung des Augenrefraktionswertes erfolgt und der gemessenen Augenrefraktionswert oder ein anhand des gemessenen Augenrefraktionswertes korrigierter Augenrefraktionswert als vorgegebener Augenrefraktionswert Verwendung findet. Durch die Berücksichtigung des vorgegebenen Augenrefraktionswerts kann für einen weiten Bereich an Augenrefraktionswerten eine Startposition erzielt werden, die bereits ein einigermaßen scharfes Bild herbeiführt oder zumindest nur einen geringen Nachfokussierungsaufwand erfordert. Dadurch kann der individuelle Zustand des jeweiligen Auges beim Einstellen der Startposition berücksichtigt werden. Die Messung des Augenrefraktionswerts kann dabei entweder ohne zuvor ausgewähltem Augenrefraktionswert erfolgen oder mit vorher vor gewähltem Augenrefraktionswert, um diesen zu korrigieren und dadurch bspw. zu verbessern.

Als Startposition findet im Rahmen des vorliegenden Verfahrens eine Fokusposition des optischen Augenbeobachtungsgeräts Verwendung, in der ein von einer vorbestimmten Ophthalmoskopierlupe aus der Gruppe von in den Beobachtungsstrahlengang des optischen Augenbeobachtungsgeräts einbringbaren Ophthalmoskopierlupen erzeugtes Luftbild ggf. unter Berücksichtigung des ausgewählten oder gemessenen Augenrefraktionswertes der Retina innerhalb eines Bereiches um die Fokusposition liegt, dessen Ausdehnung maximal der doppelten Ausdehnung, vorzugsweise maximal der anderthalbfachen Ausdehnung des Tiefenschärfenbereichs und insbesondere maximal der Ausdehnung des Tiefenschärfenbereichs des optischen Augenbeobachtungsgeräts entspricht. Dabei kann eine Fokusposition des optischen Augenbeobachtungsgeräts Verwendung finden, in der das erzeugte Luftbild der Retina eines Auges in der Mitte des Bereiches liegt, so dass der Bereich symmetrisch um die Fokusposition ist. Alternativ besteht aber auch die Möglichkeit, dass als Startposition eine Fokusposition des optischen Augenbeobachtungsgeräts Verwendung findet, in der ein von der vorbestimmten Ophthalmoskopierlupe erzeugtes Luftbild der Retina eines Auges außerhalb der Mitte des Bereiches es liegt, so dass der der Bereich nicht symmetrisch um die Fokusposition ist. Insbesondere, wenn das erzeugte Luftbild unterhalb der Mitte des Bereiches liegt, kann erreicht werden, dass die dargestellte Retina am unteren Rand des Bereiches liegt und der Bereich auch einen Bereich über der Retina, beispielsweise ein Bereich von bis zu 3 mm über der Retina, umfasst. Auf diese Weise können in der Startposition außer der Retina auch bereits Instrumentenspitzen verwendeter chirurgischer Instrumente einigermaßen scharf dargestellt werden. Zumindest kann eine solche Darstellung ohne viel Nachfokussieren erreicht werden. In noch einer weiteren Alternative kann einem Nutzer eine Einstellmöglichkeit zum Einstellen eines Abstands dargeboten werden und als Startposition dann eine Fokusposition des optischen Augenbeobachtungsgeräts Verwendung finden, in der ein von der vorbestimmten Ophthalmoskopierlupe erzeugtes Luftbild der Retina eines Auges um den eingestellten Abstand außerhalb der Mitte des Bereiches liegt. Dies ermöglicht es einem Nutzer des optischen Augenbeobachtungsgeräts, innerhalb der Grenzen der Ausdehnung des Bereiches einzustellen, wie weit oberhalb der Retina er Objekte scharf sehen möchte.

Gemäß einem zweiten Aspekt der vorliegenden Erfindung wird ein optisches Augenbeobachtungsgerät, das insbesondere ein Operationsmikroskop sein kann, mit wenigstens einer in den Beobachtungsstrahlengang einbringbaren Ophthalmoskopierlupe, und einer Fokuseinheit zum Fokussieren der Optik zur Verfügung gestellt. Die Fokuseinheit ist dazu eingerichtet, das Verfahren zum erfindungsgemäßen Einstellen einer Startposition zum Fokussieren auszuführen. Das optische Augenbeobachtungsgerät kann optional außerdem eine Messeinrichtung zum Messen des Augenrefraktionswerts umfassen. Die mit dem erfindungsgemäßen optischen Augenbeobachtungsgerät erzielbaren Eigenschaften und Vorteile ergeben sich unmittelbar aus den Eigenschaften und Vorteilen, die mit Bezug auf das erfindungsgemäße Verfahren beschrieben worden sind. Auf diese Eigenschaften und Vorteile wird verwiesen, um unnötige Wiederholungen zu vermeiden.

Weitere Merkmale, Eigenschaften und Vorteile der vorliegenden Erfindung ergeben sich aus der nachfolgenden Beschreibung von Ausführungsbeispiel unter Bezugnahme auf die beiliegenden Figuren.
- Figur 1: zeigt ein Beispiel für eine Ophthalmoskopierlupe.
- Figur 2: zeigt ein Operationsmikroskop mit einer in den Beobachtungsstrahlengang des Operationsmikroskops eingeschwenkten Ophthalmoskopierlupe.
- Figur 3: zeigt ein Beispiel für ein Hauptobjektiv mit variabler Objektschnittweite.
- Figur 4: zeigt schematisch den Fokussierbereich eines Operationsmikroskops und die Lagen von Zwischenbildebenen für zwei verschiedene Ophthalmoskopierlupen innerhalb des Fokussierbereichs.
- Figur 5: zeigt anhand eines Ablaufdiagramms das Einstellen einer Startposition für das Fokussieren eines Operationsmikroskops, wenn eine Ophthalmoskopierlupe in den Beobachtungsstrahlengang eingebracht worden ist.
- Figur 6: zeigt eine Variante des Ermittelns einer geeigneten Startposition.
- Figur 7: zeigt eine Anpassung der Startposition.

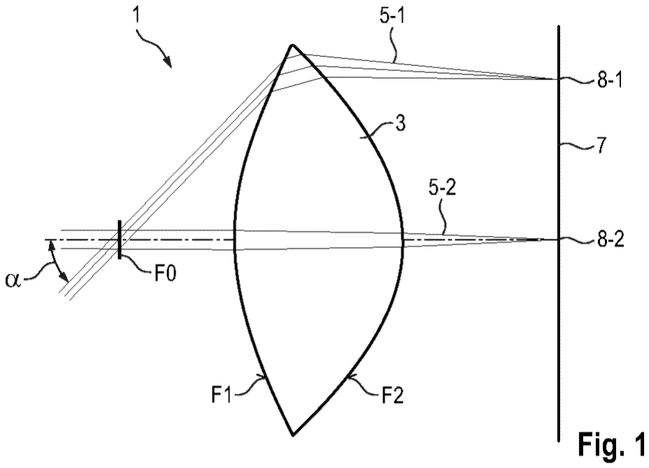

Figur 1 zeigt ein Beispiel für eine Ophthalmoskopierlupe, wie sie bei einem optischen Augenbeobachtungsgerät zum Einsatz kommen kann. In diesem Beispiel besteht die Ophthalmoskopierlupe 1 lediglich aus einer einzigen Linse 3 mit einer ersten Linsenfläche F1, die bei Gebrauch der Ophthalmoskopierlupe 1 dem Auge (nicht dargestellt) zuzuwenden ist, und einer zweiten Linsenfläche F2, die bei Gebrauch der Ophthalmoskopierlupe 1 vom Auge abgewandt ist. Die Fläche F0 stellt eine gedachte Fläche dar, die von allen Abbildungsstrahlenbündeln durchlaufen wird. In der Praxis entspricht diese Fläche der im Rahmen der Untersuchung bzw. Behandlung des Auges beleuchteten Pupille.

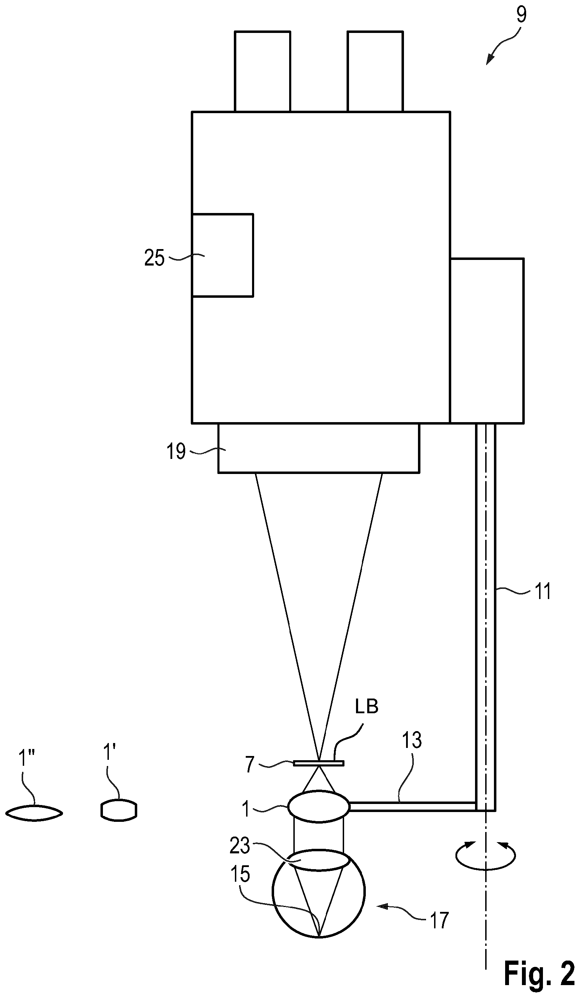

Die Ophthalmoskopierlupe 1 hat eine große Brechkraft, die im vorliegenden Beispiel 128 Dioptrien beträgt, und ist so ausgestaltet, dass sie ein ebenes Luftbild (LB) der Retina in einer Zwischenbildebene 7 erzeugt (siehe Figur 2). Mit anderen Worten, die Brennpunkte 8-1, 8-2 der von der Retina ausgehenden Strahlenbündel 5-1, 5-2 werden alle auf die Zwischenbildebene 7 fokussiert. Die Ophthalmoskopierlupe 1 des vorliegenden Beispiels kann damit in der Zwischenbildebene ein Objektfeld mit einem halben Öffnungswinkel α abbilden, wobei α in der Regel 45° oder mehr beträgt. Das von der Ophthalmoskopierlupe 1 in der Zwischenbildebene 7 erzeugte Luftbild (LB) kann dann mit dem optischen Augenbeobachtungsgerät beobachtet werden. Andere Ophthalmoskopierlupen haben geringere Brechzahlen, beispielsweise 60 Dioptrien, und bilden Objektfelder mit kleineren halben Öffnungswinkeln α ab. Eine Ophthalmoskopierlupe mit 60 Dioptrien bildet typischerweise ein Objektfeld mit einem halben Öffnungswinkel α zwischen 20° und 25° ab. Dabei gilt, dass die Brechkraft der Ophthalmoskopierlupe 1 umso größer sein muss, je größer der halbe Öffnungswinkel α des abgebildeten Objektfeldes sein soll.

Anhand von Figur 2 wird nachfolgend ein exemplarisches Ausführungsbeispiel für ein optisches Augenbeobachtungsgerät mit einer in den Beobachtungsstrahlengang einbringbaren Ophthalmoskopierlupe 1 beschrieben. Im exemplarischen Ausführungsbeispiel ist das optische Augenbeobachtungsgerät ein Operationsmikroskop 9. Es kann aber auch eine Spaltlampe sein. Die Ophthalmoskopierlupe 1 ist im vorliegenden Ausführungsbeispiel an einem Schwenksystem 11 angeordnet, mit dem sie in den Beobachtungsstrahlengang des optischen Augenbeobachtungsgeräts, also im vorliegenden exemplarischen Ausführungsbeispiel in den Beobachtungsstrahlengang des Operationsmikroskops 9, eingeschwenkt werden kann.

Der Schwenkarm 11 ist dabei so ausgestaltet, dass unterschiedliche Ophthalmoskopierlupen 1 einer Gruppe von Ophthalmoskopierlupen1, 1', 1" an einer Halterung 13 des Schwenkarms 11 befestigt werden können, sodass je nach Bedarf eine andere Ophthalmoskopierlupe 1 in den Strahlengang eingeschränkt werden kann. Alternativ kann der Schwenkarm 11 auch mehrere Halterungen 13 aufweisen, die jeweils mit einer unterschiedlichen Ophthalmoskopierlupe 1, 1', 1" versehen sind und durch unterschiedliche Schwenkstellungen des Schwenkarms 11 in den Beobachtungsstrahlengang des Operationsmikroskops 9 eingebracht werden können.

Mittels der in den Beobachtungsstrahlengang des Operationsmikroskops 9 eingeschwenkten Ophthalmoskopierlupe 1 wird ein Luftbild (LB) der Retina 15 des Patientenauges 17 in einer Zwischenbildebene 7 erzeugt. Mittels des Operationsmikroskops 9 wird dann das in der Zwischenbildebene 7 erzeugte Luftbild (LB) vergrößert betrachtet.

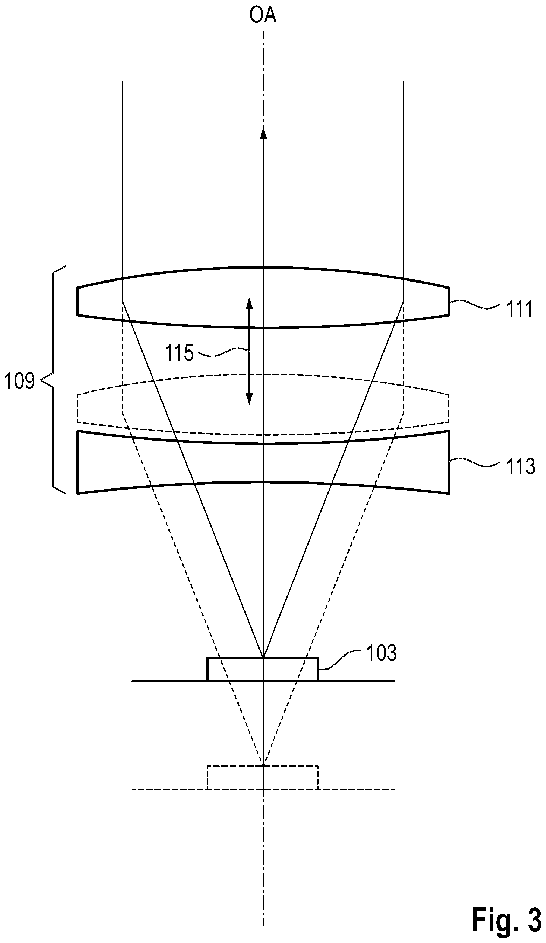

Ein Operationsmikroskop 9 ist typischerweise ergonomisch für einen Arbeitsabstand zum Auge 17 eingerichtet, in dem es den vorderen Augenabschnitt 23 scharf abbildet. Wenn nun eine Ophthalmoskopierlupe 1 in den Strahlengang eingeschwenkt wird, muss das Operationsmikroskop 9 statt dem vorderen Augenabschnitt 23 das von der Ophthalmoskopierlupe 1 in der Zwischenbildebene 7 gebildete Luftbild (LB) scharf abbilden. Im vorliegenden exemplarischen Ausführungsbeispiel weist das Operationsmikroskop 9 ein Hauptobjektiv mit variabler Objektschnittweite auf. Dadurch besteht die Möglichkeit, die Objektschnittweite des Hauptobjektivs 19 unter Beibehaltung der Arbeitsposition des Operationsmikroskops 9 in Bezug auf das Auge 17 so zu verändern, dass von einer Fokussierung auf den vorderen Augenabschnitt 23 auf eine Fokussierung auf das in der Zwischenbildebene 7 befindliche Luftbild (LB) umgestellt wird. Diese Vorgehensweise wird als "Innenfokussierung" bezeichnet. Ein Hauptobjektiv 109 mit variabler Objektschnittweite wird nachfolgend mit Bezug auf Figur 3 beschrieben, die ein solches Hauptobjektiv 109 schematisch darstellt.

Das Hauptobjektiv 109 mit variabler Objektschnittweite umfasst ein Positivglied 111, also ein optisches Element mit positiver Brechkraft, das in Figur 3 schematisch als Konvexlinse dargestellt ist. Darüber hinaus umfasst das Hauptobjektiv 109 mit variabler Objektschnittweite ein Negativglied 113, also ein optisches Element mit negativer Brechkraft, das in Figur 3 schematisch als Konkavlinse dargestellt ist. Das Negativglied 113 befindet sich zwischen dem Positivglied 111 und dem Objektfeld 103. Im dargestellten Hauptobjektiv 109 mit variabler Objektschnittweite ist das Negativglied 113 fix angeordnet, wohingegen das Positivglied 111 wie durch den Doppelpfeil 115 angedeutet entlang der optischen Achse OA verschiebbar angeordnet ist. Wenn das Positivglied 111 in die in Figur 3 gestrichelt dargestellte Position verschoben wird, verlängert sich die Objektschnittweite, so dass sich der Arbeitsabstand des Operationsmikroskops 9 vom Objektfeld 103 ändert. Obwohl in Figur 3 das Positivglied 111 verschiebbar ausgestaltet ist, besteht grundsätzlich auch die Möglichkeit, das Negativglied 113 statt des Positivglieds 111 entlang der optischen Achse OA bewegbar anzuordnen. Das Negativglied 113 bildet jedoch häufig die Abschlusslinse des Hauptobjektivs 109 mit variabler Objektschnittweite. Ein feststehendes Negativglied 113 bietet daher den Vorteil, dass das Innere des Operationsmikroskops 9 leichter gegen äußere Einflüsse abgedichtet werden kann. Weiterhin sei angemerkt, dass, obwohl das Positivglied 111 und das Negativglied 113 in Figur 3 lediglich als Einzellinsen dargestellt sind, jedes dieser Glieder statt in Form einer Einzellinse auch in Form einer Linsengruppe oder eines Kitglieds realisiert sein kann, bspw. um das Hauptobjektiv 109 mit variabler Objektschnittweite achromatisch oder apochromatisch auszubilden.

Sofern nach dem Einstellen der Startposition eine weitere Fokussierung nötig ist, kann diese manuell erfolgen, indem der Nutzer die Objektschnittweite händisch gesteuert einstellt. Alternativ besteht die Möglichkeit eines Autofokus, bei dem diese weitere Fokussierung nach dem Einstellen der Startposition ohne Zutun des Nutzers automatisiert erfolgen kann.

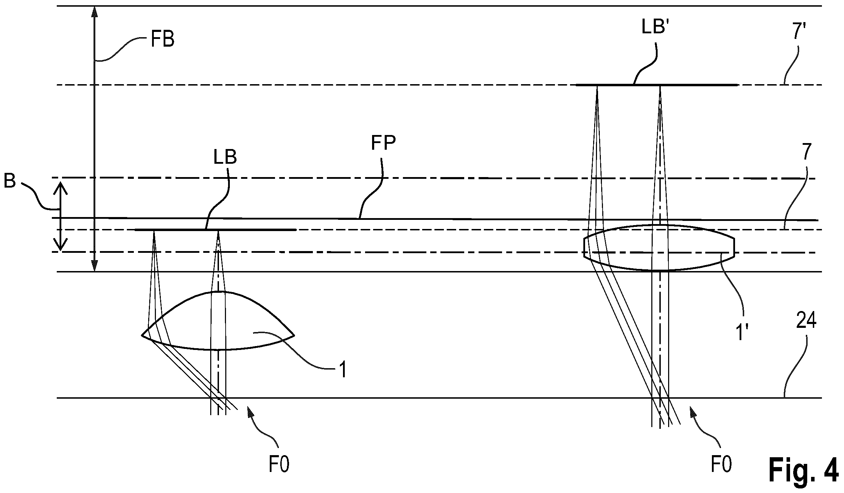

Um die Fokussierung des Operationsmikroskops 9 auf das in der Zwischenbildebene 7 befindliche Luftbild (LB) zu erleichtern, wird das Operationsmikroskop 9 im vorliegenden exemplarischen Ausführungsbeispiel bei Einschwenken einer Ophthalmoskopierlupe 1 in eine Startposition gebracht, von der aus die Fokussierung manuell oder automatisiert erfolgen kann. Die Startposition soll dabei den zum Fokussieren benötigten Verstellweg verringern und im Idealfall bereits ein annähernd fokussiertes Bild liefern. Jede der unterschiedlichen Ophthalmoskopierlupen 1, 1', 1" bildet die Retina 15 jedoch in eine andere Zwischenbildebene 7 ab. Mit anderen Worten, der Abstand der Zwischenbildebene vom objektseitigen Linsenscheitel des Hauptobjektivs 19 des Operationsmikroskops 9 hängt von der verwendeten Ophthalmoskopierlupe 1, 1', 1" ab. Diese Situation ist in Figur 4 schematisch am Beispiel zweier Ophthalmoskopierlupen 1, 1' dargestellt. Neben den beiden Ophthalmoskopierlupen 1, 1' zeigt die Figur den Fokussierbereich FB, innerhalb dessen eine Fokussierung möglich ist, die Ebene 24, in welcher die Flächen F0 der jeweiligen Ophthalmoskopierlupe 1, 1' liegen und die im Wesentlichen dem Ort der Augenpupille entspricht, sowie die Zwischenbildebenen 7, 7' der beiden Ophthalmoskopierlupen 1, 1'. Eine der beiden Ophthalmoskopierlupen 1 weist dabei eine hohe Brechkraft auf, beispielsweise 128 Dioptrien. Die andere Ophthalmoskopierlupe 1' weist dagegen eine niedrigere Brechkraft auf, beispielsweise 60 Dioptrien. Die unterschiedlichen Brechtkräfte der beiden Ophthalmoskopierlupen 1, 1' führen dabei dazu, dass die zugehörigen Zwischenbildebenen 7, 7' an deutlich unterschiedlichen Positionen innerhalb des Fokussierbereichs FB liegen.

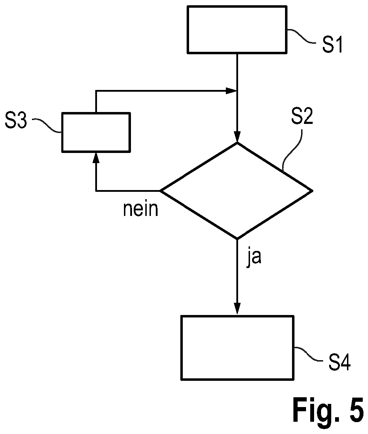

Das Einstellen einer Startposition für das Fokussieren des Operationsmikroskops 9, wenn eine Ophthalmoskopierlupe 1 in den Beobachtungsstrahlengang eingebracht worden ist, wird nachfolgend mit Bezug auf Figur 5 beschrieben. Die Figur zeigt anhand eines Ablaufdiagramm die beim Einstellen der Startposition durchgeführten Schritte.

Nachdem das Verfahren in Schritt S1 gestartet worden ist, erfolgt in Schritt S2 eine Abfrage, ob eine Ophthalmoskopierlupe 1 in den Beobachtungsstrahlengang des Operationsmikroskops 9 eingeschwenkt worden ist. Ist dies nicht der Fall, schreitet das Verfahren zu Schritt S3 fort, in dem eine vorbestimmte Zeitspanne abgewartet wird, bevor das Verfahren zu Schritt S2 zurückkehrt und erneut eine Abfrage vornimmt, ob eine Ophthalmoskopierlupe 1 in den Beobachtungsstrahlengang des Operationsmikroskops 9 eingeschwenkt worden ist. Sobald diese Abfrage bejaht wird, schreitet das Verfahren zu Schritt S4 fort, in welchem eine Einstelleinheit die Startposition für das Fokussieren des Operationsmikroskops 9 automatisch einstellt, von der aus dann eine weitere Fokussierung vorgenommen werden kann, sofern dies nötig ist. Im einfachsten Fall ist als Startposition eine Fokusposition FP des Operationsmikroskops 9 hinterlegt, in der die Zwischenbildebene 7 einer vorbestimmten Ophthalmoskopierlupe 1 aus der Gruppe von Ophthalmoskopierlupen 1, 1', 1" innerhalb eines Bereiches B um die Fokusposition FP liegt, dessen Ausdehnung im vorliegenden exemplarischen Ausführungsbeispiel der Ausdehnung des Tiefenschärfenbereiches des Operationsmikroskops 9 entspricht. Die Startposition ist im Falle einer Außenfokussierung dabei diejenige Position, die das Operationsmikroskops 9 einnehmen muss, damit die Objektschnittweite des Hauptobjektivs 19 gleich dem Abstand des Linsenscheitels des Hauptobjektivs 19 von der Zwischenbildebene 7 der vorbestimmten Ophthalmoskopierlupe 1 ist. Im Falle einer Innenfokussierung gibt die Startposition diejenige Stellung der Linsen des Hauptobjektivs mit variabler Objektschnittweite relativ zueinander an, für die die Objektschnittweite des Hauptobjektivs 19 gleich dem Abstand des objektseitigen Linsenscheitels des Hauptobjektivs 19 von der Zwischenbildebene 7 der vorbestimmten Ophthalmoskopierlupe 1 ist.

Im vorliegenden exemplarischen Ausführungsbeispiel ist die vorbestimmte Ophthalmoskopierlupe 1 die Ophthalmoskopierlupe mit der größten Brechkraft, im vorliegenden exemplarischen Ausführungsbeispiel also die Ophthalmoskopierlupe mit 128 Dioptrien. Diese Ophthalmoskopierlupe liefert den besten Überblick über die Retina und wird deshalb häufig als erste in den Beobachtungsstrahlengang des Operationsmikroskops 9 eingebracht. Wenn der Abstand der Zwischenbildebene dieser Ophthalmoskopierlupe 1 vom objektseitigen Linsenscheitel des Hauptobjektivs 19 zum Einstellen der Startposition für das Fokussieren herangezogen wird, kann mit der Startposition häufig bereits ein gut fokussiertes Bild erzielt werden, zumindest ist aber nur eine geringfügige Nachfokussierung nötig. Selbstverständlich besteht auch die Möglichkeit, die Startposition für das Fokussieren an die Lage der Zwischenbildebene 7 einer anderen Ophthalmoskopierlupe als der Ophthalmoskopierlupe mit der größten Brechkraft zu knüpfen. In der Regel wird die Startposition an die Lage der Zwischenbildebene derjenigen Ophthalmoskopierlupe aus einer Gruppe von zur Verfügung stehenden Ophthalmoskopierlupen 1, 1', 1" geknüpft, die am häufigsten oder als erstes in der Behandlung Verwendung findet, damit die Zahl der Fälle, für die die Startposition bereits ein gut fokussiertes Bild liefert oder zumindest nur eine geringfügige Nachfokussierung erfordert, möglichst groß ist.

In dem Verfahren, wie es mit Bezug auf Figur 5 beschrieben worden ist, wird von einem emmetropen (rechtsichtigen) Auge ausgegangen. Dies ist insofern gerechtfertigt, als dass die meisten Hinterabschnittsoperationen am Auge eine Entfernung des Glaskörpers (Vitrektomie) beinhalten, die mit sehr hoher Wahrscheinlichkeit zu einem Katarakt führt. Daher erfolgt vor einer Vitrektomie in der Regel eine Kataraktoperation, bei der die natürliche Augenlinse entfernt und stattdessen eine intraokulare Linse (IOL) eingesetzt wird. Die eingesetzten intraokularen Linsen führen in der Regel zu einem rechtsichtigen Auge oder gegebenenfalls zu einem leicht kurzsichtigen Auge, das entweder keine Korrektur benötigt oder lediglich eine leichte Korrektur zwischen 0 und - 2 Dioptrien. Bei einer eingeschwenkten Ophthalmoskopierlupe 1 mit einer Brechkraft liegen die Zwischenbildebene für ein Auge, das eine Korrektur von 0 Dioptrien benötigt und die Zwischenbildebene für ein Auge, dass eine Korrektur von -2 Dioptrien benötigt, nur um weniger als 1 mm voneinander entfernt. Im Falle einer Ophthalmoskopierlupe mit einer Brechkraft von 128 Dioptrien haben diese Zwischenbildebenen einen Abstand von 0,12 mm. Im Falle einer Ophthalmoskopierlupe mit einer Brechkraft von 60 Dioptrien beträgt der Abstand 0,52 mm. Somit ist insbesondere dann, wenn die Startposition an eine Ophthalmoskopierlupe mit hoher Brechkraft geknüpft ist, bspw. an die Ophthalmoskopierlupe mit 128 Dioptrien, die Wahrscheinlichkeit groß, mit der Startposition bereits ein hinreichend fokussiertes Bild zu erhalten oder ein Bild, das lediglich wenig nachfokussiert werden muss.

In einer alternativen Ausgestaltung des Verfahrens ermöglicht dieses eine Auswahl derjenigen Ophthalmoskopierlupe 1, die in den Beobachtungsstrahlengang des Operationsmikroskops 9 eingeschwenkt werden soll, aus einer Anzahl zur Verfügung stehender Ophthalmoskopierlupen 1, 1', 1". Außerdem ermöglicht das Verfahren in dieser Ausgestaltung auch eine Auswahl der Brechkraft der Augenlinse des zu untersuchenden Auges 17 aus einer Liste von Brechkräften. Im Operationsmikroskop 9 ist dann zu jeder auswählbaren Kombination von zur Verfügung stehenden Ophthalmoskopierlupen 1, 1', 1" und Brechkraft der Augenlinse eine eigene Startposition zugeordnet. Je nach ausgewählter Ophthalmoskopierlupe und ausgewählter Brechkraft der Augenlinse wird dann die Fokusposition FP des Operationsmikroskops 9 in die der Kombination aus ausgewählter Ophthalmoskopierlupe und ausgewählter Brechkraft der Augenlinse zugeordnete Startposition gebracht.

Um die Auswahlen der Ophthalmoskopierlupe 1 zu ermöglichen, schließt sich an den Start S1 des Verfahrens ein Schritt S11 an, in dem diejenigen in den Beobachtungsstrahlengang des Operationsmikroskops 9 einschwenkbaren Ophthalmoskopierlupen 1, 1', 1" angeboten werden, für die Startpositionen hinterlegt sind, um eine von ihnen auszuwählen. Beispielsweise können die einschwenkbaren Ophthalmoskopierlupen, für die Startpositionen hinterlegt sind, auf einem Display dargestellt werden, welches dem Operationsmikroskops 9 zugeordnet ist. Mittels einer Auswahleinheit, beispielsweise einer Tastatur, eines Joysticks, eines Touchpads, des Displays, falls das Display ein Touchscreen ist, etc. kann der Nutzer des Operationsmikroskops 9 in Schritt S12 dann diejenige Ophthalmoskopierlupe 1 auswählen, die in den Beobachtungsstrahlengang des Operationsmikroskops 9 eingeschwenkt werden soll. Dabei können im Operationsmikroskop 9 insbesondere für alle in den Beobachtungsstrahlengang einschwenkbaren Ophthalmoskopierlupen 1, 1', 1" Startpositionen hinterlegt sein, und es können alle diese in den Beobachtungsstrahlengang ein schwenkbaren Ophthalmoskopierlupen 1, 1', 1" zur Auswahl angeboten werden.

Im nächsten Schritt S13 wird dem Nutzer die Möglichkeit geboten, aus einer Anzahl von Augenrefraktionswerten denjenigen Augenrefraktionswert auszuwählen, der dem Patientenauge entspricht oder dem Patientenauge am nächsten kommt. Um diese Auswahl zu ermöglichen kann dasselbe Display und dieselbe Auswahleinheit wie beim Auswählen der Ophthalmoskopierlupe Verwendung finden. Der Nutzer kann dann in Schritt S14 den dem Patientenauge 17 entsprechenden Augenrefraktionswert auswählen oder denjenigen Augenrefraktionswert auswählen, der dem Patientenauge 17 am nächsten kommt.

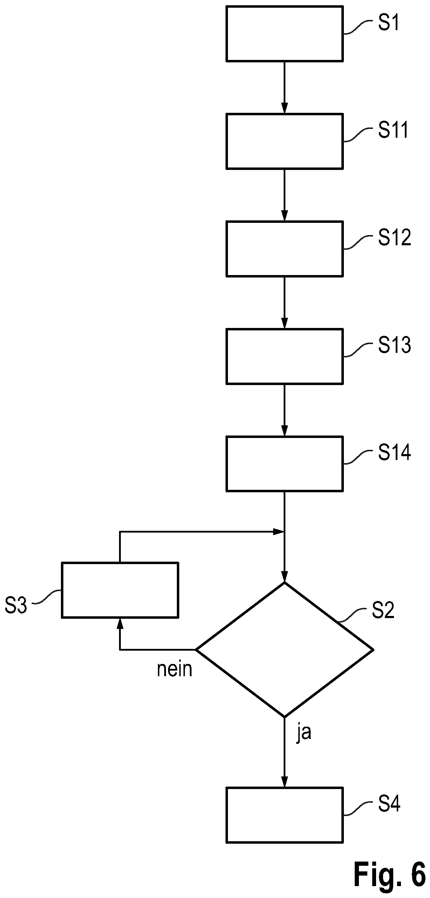

Nachdem die Ophthalmoskopierlupe 1 ausgewählt worden ist und der Augenrefraktionswert des Patientenauges 17 bestimmt worden ist, schreitet das Verfahren zu Schritt S2 fort. Hinsichtlich der Schritte S2 und S3 unterscheidet sich das Verfahren gemäß Figur 6 nicht vom Verfahren gemäß Figur 5.

Im Schritt S4 stellt eine Einstelleinheit dann automatisch eine Startposition für das Fokussieren des Operationsmikroskops 9 ein, von der aus die weitere Fokussierung vorgenommen werden kann. Dabei ist im Operationsmikroskop 9 jeder auswählbaren Kombination aus Ophthalmoskopierlupe 1, 1', 1" und Augenrefraktionswert eine eigene Startposition für die Fokusposition FP des Operationsmikroskops 9 zugeordnet. Eine Einstelleinheit stellt dann je nach Kombination aus ausgewählter Ophthalmoskopierlupe1, 1', 1" und ausgewähltem Augenrefraktionswert die der Kombination zugeordnete Startposition ein. Die Zuordnung kann im Operationsmikroskop 9 in Form einer Nachschlagtabelle hinterlegt sein. Alternativ besteht die Möglichkeit, eine Formel oder einen funktionalen Zusammenhang im Operationsmikroskop 9 zu hinterlegen, mit der bzw. dem die Startposition, d. h. im Falle einer Außenfokussierung diejenige Position, die das Operationsmikroskops 9 einnehmen muss, damit die Objektschnittweite des Hauptobjektivs 19 gleich dem Abstand des Linsenscheitels des Hauptobjektivs 19 von der Zwischenbildebene 7 der ausgewählten Ophthalmoskopierlupe 1 ist bzw. im Falle einer Innenfokussierung diejenige Stellung der Linsen des Hauptobjektivs mit variabler Objektschnittweite relativ zueinander, für die die Objektschnittweite des Hauptobjektivs 19 gleich dem Abstand des objektseitigen Linsenscheitels des Hauptobjektivs 19 von der Zwischenbildebene 7 der vorbestimmten Ophthalmoskopierlupe 1 ist, in Abhängigkeit von der ausgewählten Ophthalmoskopierlupe 1 und dem ausgewählten Augenrefraktionswert ausgerechnet werden kann.

Wenn im Operationsmikroskop 9 eine Formel oder ein funktionaler Zusammenhang hinterlegt ist, die es ermöglicht, die Startposition in Abhängigkeit von der ausgewählten Ophthalmoskopierlupe 1 und dem einem Augenrefraktionswert auszurechnen, kann statt der Auswahl des Augenrefraktionswerts aus einer Anzahl vorgegebener Augenrefraktionswerte dem Nutzer auch die Möglichkeit geboten werden, einen Augenrefraktionswert als Zahlenwert einzugeben. Der Zahlenwert kann beispielsweise vorab durch eine Messung am Patientenauge 17 ermittelt werden. Die Messung der Augenrefraktion des Patientenauge 17 kann dabei mit allen gängigen Methoden erfolgen. Beispiele hierfür sind das Messen der Augenrefraktion mittels optischer Kohärenztomografie (OCT), mittels Ultraschall, etc. Als weitere Alternative besteht die Möglichkeit, dass dem Operationsmikroskop 9 eine Vorrichtung zum Messen des Augenrefraktionswerts zugeordnet ist, und dass vor dem Einschwenken der Ophthalmoskopierlupe 1 in den Beobachtungsstrahlengang eine Messung des Augenrefraktionswertes am Patentenauge 17 vorgenommen wird, der dann automatisch an das Operationsmikroskop 9 weitergegeben wird. Eine automatische Vorgabe des Augenrefraktionswerts kann im Rahmen der Implantation einer intraokularen Linse auch vom IOL-Master an das Operationsmikroskop 9 erfolgen. In den Fällen der automatischen Vorgabe des Augenrefraktionswerts braucht der Nutzer keinen Augenrefraktionswert einzugeben oder auszuwählen. Es besteht aber auch die Möglichkeit, eine Messung des Augenrefraktionswerts durchzuführen, nachdem vom Nutzer ein Augenrefraktionswert aus einer Liste von Augenrefraktionswerten ausgewählt worden ist oder nachdem eine intraokulare Linse eingesetzt worden ist, um den ausgewählten bzw. den vom IOL-Master erhaltenen Augenrefraktionswert zu prüfen und ggf. zu präzisieren. Eine Refraktionsmesseinheit 25, etwa in Form einer OCT-Einheit oder einer Ultraschalleinheit, kann dabei auch in das Operationsmikroskops 9 integriert sein, was in Figur 2 schematisch dargestellt ist.

In dem in Figur 6 dargestellten Verfahren wird sowohl die Ophthalmoskopierlupe 1, welche in den Beobachtungsstrahlengang des Operationsmikroskops 9 eingebracht werden soll, ausgewählt, als auch der Augenrefraktionswert des Patientenauge 17 bestimmt. In Abwandlungen des Verfahrens kann aber auch entweder nur die Ophthalmoskopierlupe 1, welche in den Beobachtungsstrahlengang des Operationsmikroskops 9 eingebracht werden soll, ausgewählt oder nur der Augenrefraktionswert des Patientenauges 17 bestimmt werden. Im ersteren Fall wird dann typischerweise von einem rechtsichtigen Patientenauge 17 ausgegangen, im letzteren Fall findet als Startposition typischerweise eine Fokusposition FP des Operationsmikroskops 9 Verwendung, welche auf derjenigen Ophthalmoskopierlupe 1 basiert, die am häufigsten oder als erstes in der Behandlung zum Einsatz kommt.

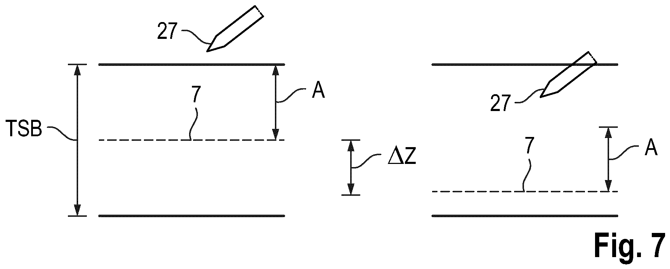

Im Rahmen des beschriebenen Verfahrens kann als Startposition eine Fokusposition FP des Operationsmikroskops 9 Verwendung finden, in der die Fokusebene 7 der Ophthalmoskopierlupe 1, und damit das von der vorbestimmten Ophthalmoskopierlupe 1 erzeugte Luftbild (LB) der Retina 15, in der Mitte des Tiefenschärfenbereiches TSB des Operationsmikroskops 9 liegt, wie dies schematisch in Figur 7 dargestellt ist. Alternativ besteht die Möglichkeit, die Fokusposition FP des Operationsmikroskops 9 für die Startposition so zu wählen, dass die Zwischenbildebene 7 um einen Wert Δz aus der Mitte des Tiefenschärfenbereich TSB des Operationsmikroskops 9 in den unteren Abschnitt des Tiefenschärfenbereiches TSB verschoben ist. Auch dies ist in Figur 7 dargestellt. Das Einstellen der Fokusposition FP derart, dass die Zwischenbildebene 7 im unteren Abschnitt des Tiefenschärfenbereichs TSB verschoben ist, ermöglicht es, eine Instrumentenspitze 27 in einem Abstand A von der Retina 15 bereits fokussiert oder zumindest mit nur einer geringen Unschärfe zu sehen, wenn sich die Fokusposition FP des Operationsmikroskops 9 in der Startposition befindet. Dabei kann dem Operationsmikroskop 9 auch eine Einstelleinrichtung zugeordnet sein, mit der sich die Verschiebung Δz vorgeben lässt. Eine große Verschiebung Δz birgt jedoch das Risiko, dass die Fokussierung der Retina 15 in der Startposition für die Fokussierung des Operationsmikroskops 9 schlechter ist als ohne Verschiebung Δz oder mit einer kleinen Verschiebung Δz.

Die vorliegende Erfindung wurde anhand exemplarischer Ausführungsbeispiele zu Erläuterungszwecken im Detail beschrieben. Ein Fachmann erkennt anhand der Beschreibung jedoch, dass er im Rahmen der Erfindung, wie sie in den beiliegenden Ansprüchen definiert ist, von den exemplarischen Ausführungsbeispielen abweichen kann. Beispielsweise kann die Ophthalmoskopierlupe aus mehr als einer Linse bestehen, beispielsweise zwei oder drei Linsen. Die Erfindung soll daher ausschließlich durch die beigefügten Ansprüche, nicht jedoch durch die exemplarischen Ausführungsbeispiele beschränkt sein. Zudem muss die Startposition nicht zwingend so gewählt sein, dass die Zwischenbildebene der vorbestimmten Ophthalmoskopierlupe innerhalb des Tiefenschärfenbereiches des Operationsmikroskops liegt. Es ist ausreichend, wenn die Startposition bereits ein halbwegs scharfes Bild liefert. Dies kann aber bereits erreicht werden, wenn die Startposition so gewählt ist, dass die Zwischenbildebene der vorbestimmten Ophthalmoskopierlupe innerhalb eines Bereiches von der als Fokusposition verwendeten Startposition liegt, dessen Ausdehnung maximal dem doppelten, vorzugsweise maximal dem anderthalbfachen, des Tiefenschärfenbereiches des Operationsmikroskops entspricht.

### Bezugszeichenliste

- 1: Ophthalmoskopierlupe
- 1': Ophthalmoskopierlupe
- 1": Ophthalmoskopierlupe
- 3: Linse
- 5-1: Strahlenbündel
- 5-2: Strahlenbündel
- 7: Zwischenbildebene
- 7': Zwischenbildebene
- 8-1: Brennpunkt
- 8-2: Brennpunkt
- 9: Operationsmikroskop
- 11: Schwenkarm
- 13: Halterung
- 15: Retina
- 17: Patientenauge
- 19: Hauptobjektivs
- 23: vorderer Augenabschnitt
- 24: Ebene
- 25: Refraktionsmesseinheit
- 27: Instrumentenspitze
- 103: Objektfeld
- 109: Hauptobjektivs mit variabler Objektschnittweite
- 111: Positivglied
- 113: Negativglied
- 115: Verschiebung
- A: Abstand
- F0: gedachte Fläche
- F1: Linsenfläche
- F2: Linsenfläche
- FB: Fokussierbereich
- S1: Start
- S2: Detektieren, ob eine Ophthalmoskopierlupe eingeschwenkt ist
- S3: Warten
- S4: Bringen der Fokusposition des Operationsmikroskops in die Startposition
- S11: Anbieten der in den Beobachtungsstrahlengang des Operationsmikroskops ein schwenkbaren Ophthalmoskopierlupe
- S12: Auswahl der Ophthalmoskopierlupe
- S13: Anbieten einer Auswahl an Augenrefraktionswerten
- S14: Auswahl eines Augenrefraktionswerts
- B: Bereich
- FB: Fokussierbereich
- FP: Fokusposition
- LB: Luftbild
- TSB: Tiefenschärfenbereich

## Patentansprüche

1. Verfahren zum Einstellen einer Startposition für das Fokussieren eines optischen Augenbeobachtungsgeräts (9) bei Einbringen einer Ophthalmoskopierlupe (1) in den Beobachtungsstrahlengang des optischen Augenbeobachtungsgeräts (9), in dem
- das Einbringen der Ophthalmoskopierlupe (1) in den Beobachtungsstrahlengang detektiert wird,
- die Fokusposition (FP) des optischen Augenbeobachtungsgeräts (9) bei Detektion des Einbringens der Ophthalmoskopierlupe (1) automatisiert in eine Startposition gebracht wird;
wobei
als Startposition eine Fokusposition (FP) des optischen Augenbeobachtungsgeräts (9) Verwendung findet, in der ein von einer vorbestimmten Ophthalmoskopierlupe (1) aus der Gruppe von in den Beobachtungsstrahlengang des optischen Augenbeobachtungsgeräts (9) einbringbaren Ophthalmoskopierlupen (1, 1', 1") erzeugtes Luftbild (LB) der Retina (15) innerhalb eines Bereiches (B) um die Fokusposition (FP) liegt, dessen Ausdehnung maximal der doppelten Ausdehnung des Tiefenschärfenbereichs (TSB) des optischen Augenbeobachtungsgeräts (9) entspricht.

2. Verfahren nach Anspruch 1, in dem die Gruppe von Ophthalmoskopierlupen (1, 1', 1") wenigstens eine der folgenden Ophthalmoskopierlupen umfasst:
- die am häufigsten in der Behandlung Verwendung findende Ophthalmoskopierlupe,
- diejenige Ophthalmoskopierlupe, welche die höchste Brechkraft aufweist,
wobei eine dieser Ophthalmoskopierlupen die vorbestimmte Ophthalmoskopierlupe bildet.

3. Verfahren nach Anspruch 1 oder Anspruch 2, in dem die Gruppe von Ophthalmoskopierlupen (1, 1', 1") die als erstes in der Behandlung Verwendung findende Ophthalmoskopierlupe umfasst und diese die vorbestimmte Ophthalmoskopierlupe bildet.

4. Verfahren nach einem der Ansprüche 1 bis 3, in dem
- für eine Anzahl an Ophthalmoskopierlupen (1, 1', 1") aus der Gruppe von Ophthalmoskopierlupen (1, 1', 1") Startpositionen im optischen Augenbeobachtungsgerät gespeichert sind;
- einem Nutzer des optischen Augenbeobachtungsgeräts die Anzahl an Ophthalmoskopierlupen (1, 1', 1") zur Auswahl einer der Ophthalmoskopierlupen (1) aus dieser Anzahl an Ophthalmoskopierlupen (1, 1', 1") dargeboten werden,
- die vom Nutzer getroffene Auswahl erfasst wird und
- als Startpositionen die für die vom Nutzer ausgewählte Ophthalmoskopierlupe (1) gespeicherte Startposition Verwendung findet.

5. Verfahren nach einem der Ansprüche 1 bis 4, in dem als Startposition eine Fokusposition (FP) des optischen Augenbeobachtungsgeräts (9) Verwendung findet, in der ein von der vorbestimmten Ophthalmoskopierlupe (1) erzeugtes Luftbild (LB) der Retina (15) eines emmetropen Auges (17) innerhalb des Bereiches (B) liegt.

6. Verfahren nach einem der Ansprüche 1 bis 5, in dem
- ein Augenrefraktionswert vorgegeben wird, und
- als Startposition eine Fokusposition (FP) des optischen Augenbeobachtungsgeräts (9) Verwendung findet, in der ein von der vorbestimmten Ophthalmoskopierlupe (1) erzeugtes Luftbild (LB) der Retina (15) eines Auges (17) mit dem vorgegebenen Augenrefraktionswert innerhalb des Bereiches (B) liegt.

7. Verfahren nach Anspruch 6, in dem einem Nutzer eine Anzahl an Augenrefraktionswerten zur Auswahl eines dieser Augenrefraktionswerte als vorgegebenem Augenrefraktionswert dargeboten wird.

8. Verfahren nach einem der Ansprüche 1 bis 7, in dem als Startposition eine Fokusposition (FP) des optischen Augenbeobachtungsgeräts Verwendung findet, in der ein von der vorbestimmten Ophthalmoskopierlupe erzeugtes Luftbild (LB) der Retina (15) eines Auges in der Mitte des Bereich (B) liegt

9. Verfahren nach einem der Ansprüche 1 bis 7, in dem als Startposition eine Fokusposition (FP) des optischen Augenbeobachtungsgeräts (9) Verwendung findet, in der ein von der vorbestimmten Ophthalmoskopierlupe (1) erzeugtes Luftbild (LB) der Retina (15) eines Auges (17) außerhalb der Mitte des Bereiches (B) liegt.

10. Verfahren nach Anspruch 9, in dem als Startposition eine Fokusposition (FP) des optischen Augenbeobachtungsgeräts (9) Verwendung findet, in der ein von der vorbestimmten Ophthalmoskopierlupe (1) erzeugtes Luftbild (LB) der Retina (15) eines Auges (17) am unteren Rand des Bereichs (B) liegt.

11. Verfahren nach Anspruch 9 oder Anspruch 10, in dem
- einem Nutzer eine Einstellmöglichkeit zum Einstellen eines Abstands (Δz) dargeboten wird und
- als Startposition eine Fokusposition (FP) des optischen Augenbeobachtungsgeräts (9) Verwendung findet, in der ein von der vorbestimmten Ophthalmoskopierlupe (1) erzeugtes Luftbild (LB) der Retina (15) eines Auges (17) mit dem gemessenen Augenrefraktionswert um den eingestellten Abstand (Δz) außerhalb der Mitte des Bereiches (AB) liegt.

12. Verfahren nach einem der Ansprüche 1 bis 11, in dem als Startposition eine Fokusposition (FP) des optischen Augenbeobachtungsgeräts (9) Verwendung findet, in der das Luftbild (LB) der Retina (15) innerhalb des Tiefenschärfenbereiches (TSB) des optischen Augenbeobachtungsgeräts (9) liegt.

13. Optisches Augenbeobachtungsgerät (9) mit
- wenigstens einer in den Beobachtungsstrahlengang einbringbaren Ophthalmoskopierlupe, und
- einer Fokussiereinheit (109) zum Fokussieren der Optik, wobei die Fokussiereinheit dazu eingerichtet ist, das Verfahren zum Einstellen einer Startposition zum Fokussieren nach einem der Ansprüche 1 bis 12 auszuführen.

14. Optisches Augenbeobachtungsgerät (9) nach Anspruch 13, das außerdem eine Messeinrichtung (25) zum Messen des Augenrefraktionswerts umfasst.

15. Optisches Augenbeobachtungsgerät nach Anspruch 13 oder Anspruch 14, das als Operationsmikroskop (9) oder Spaltlampe ausgebildet ist.
